**Europäisches Patentamt**

(19) **European Patent Office**

**Office européen des brevets**

(11) Veröffentlichungsnummer: **0 108 934**
**B1**

(12) EUROPÄISCHE PATENTSCHRIFT

(45) Veröffentlichungstag der Patentschrift:
**10.12.86**

(51) Int. Cl.⁴: **C 14 C 9/02**

(21) Anmeldenummer: **83110170.4**

(22) Anmeldetag: **12.10.83**

(54) **Fettungsmittel für Leder und Pelze.**

(30) Priorität: **20.10.82 DE 3238741**

(43) Veröffentlichungstag der Anmeldung:
**23.05.84 Patentblatt 84/21**

(45) Bekanntmachung des Hinweises auf die Patenterteilung:
**10.12.86 Patentblatt 86/50**

(84) Benannte Vertragsstaaten:
**AT BE CH DE FR GB IT LI NL**

(56) Entgegenhaltungen:
**EP - A - 0 039 858**
**FR - A - 2 200 356**

**Die Akte enthält technische Angaben, die nach dem
Eingang der Anmeldung eingereicht wurden und die
nicht in dieser Patentschrift enthalten sind.**

(73) Patentinhaber: **Henkel Kommanditgesellschaft auf
Aktien, Postfach 1100 Henkelstrasse 67,
D-4000 Düsseldorf-Holthausen (DE)**

(72) Erfinder: **Willmund, Wolf-Dieter, Dr., Einsteinstrasse 9,
D-4000 Düsseldorf (DE)**
Erfinder: **Biermann, Manfred, Dr., Stooter Strasse 18,
D-4330 Mühlheim a.d. Ruhr (DE)**
Erfinder: **Baumann, Horst, Dr., Vogelwarte 17,
D-5653 Leichlingen (DE)**
Erfinder: **Friese, Hans-Herbert, Dr., Schiesshecke 53,
D-4019 Monheim (DE)**
Erfinder: **Pieper, Friedrich, Zehntenweg 10,
D-4018 Langenfeld (DE)**

## Beschreibung

Aus DE-C-22 45 077 sind Fettungsmittel für Leder oder Pelze auf der Basis von sulfonierten Chlorierungsprodukten von natürlichen oder synthetischen höheren Fettsäuren oder Fettsäureestern in Form ihrer Alkali-, Ammonium- oder Aminsalze bekannt, die dadurch gekennzeichnet sind, dass sie aus solchen sulfonierten Chlorierungsprodukten bestehen, die durch Chlorieren von höheren Fettsäuren oder von Estern höherer Fettsäuren der Kettenlängen $C_8$ bis $C_{24}$ bis zu einem Chlorgehalt von 20 bis 45 Gewichtsprozent, wobei die Chlorierungsprodukte im wesentlichen keine olefinischen Doppelbindungen mehr enthalten, und nachfolgende Sulfonierung mit $SO_3$ bis zu einem Gehalt von 40 bis 100 Molprozent $SO_3$, bezogen auf Chlorierungsprodukt, erhalten worden sind.

Aus DE-A-30 18 176 sind weiterhin Fettungsmittel für Leder oder Pelze auf der Basis von sulfonierten Chlorierungsprodukten von natürlichen oder synthetischen höheren Fettsäuren oder Fettsäureestern in Form ihrer Alkali-, Ammonium- oder Aminsalze bekannt, die dadurch gekennzeichnet sind, dass sie im wesentlichen aus solchen sulfonierten Chlorierungsprodukten bestehen, die durch Sulfochlorieren von höheren Fettsäuren oder von Estern höherer Fettsäuren der Kettenlängen $C_8$ bis $C_{24}$ mittels Chlor und $SO_2$ ggf. unter UV-Bestrahlung bei 20–90 °C bis zu einem Gehalt an gebundenem Chlor von 5–30 Gewichtsprozent und einem Gehalt an $SO_2Cl$-Gruppen von 1–20 Gewichtsprozent, wobei das Verhältnis von Chloratomen zu $SO_2Cl$-Gruppen 0,7:1 bis 70:1 beträgt, und nachfolgende Verseifung erhalten worden sind.

Es wurde nun gefunden, dass sich die bekannten Verfahren zur Herstellung derartiger Fettungsmittel weiterhin verbessern lassen, sofern man bei der Herstellung der sulfonierten Chlorierungsprodukte von höheren Fettsäure- oder Fettsäureestergemischen ausgeht, die ungesättigte Anteile enthalten. In diesem Falle wird zunächst eine Chlorierung zur Absättigung der Doppelbindungen, und danach eine Sulfochlorierung mit Chlor und $SO_2$ durchgeführt. Die nachfolgende Verseifung führt zu den gewünschten Fettungsmitteln.

Gegenstand der Erfindung ist somit ein Verfahren zur Herstellung von Fettungsmitteln für Leder und Pelze durch Umsetzung von Gemischen gesättigter und ungesättigter Fettsäuren mit Kettenlängen im Bereich von $C_8$ bis $C_{24}$ oder Estern solcher Fettsäuren mit Chlor und $SO_2$ durch

a) Sulfochlorierung solcher Fettsäuren bzw. deren Ester mittels Chlor und $SO_2$ bei 20 bis 90 °C, gegebenenfalls unter UV-Bestrahlung, und

b) anschliessender Verseifung der erhaltenen Produkte zu den entsprechenden Alkali-, Ammonium- oder Aminsalzen, dadurch gekennzeichnet, dass man

c) solche Fettsäuren bzw. deren Ester vor der Sulfochlorierung bis zur Absättigung der Doppelbindungen bei Temperaturen bis maximal 30 °C, gewünschtenfalls bei Temperaturen zwischen 20 und 25 °C, chloriert, wobei

d) die nach Chlorierung und Sulfochlorierung erhaltenen Produkte einen Gehalt an organisch gebundenem Chlor im Bereich von 5 bis 30 Gew.-% und einen Gehalt an $SO_2Cl$-Gruppen im Bereich von 1 bis 20 Gew.-% sowie ein Verhältnis von organisch gebundenen Chloratomen zu $SO_2Cl$-Gruppen im Bereich von 0,7:1 bis 70:1 aufweisen.

Das neue Verfahren führt zu einer besseren Ausnutzung der eingesetzten Gase, d.h. zu geringeren Chlor- und $SO_2$-Verlusten im Abgas und damit verbunden zu einem höheren Sulfonierungsgrad der eingesetzten Fettstoffe bzw. zu geringerem Verbrauch an Chlor und $SO_2$.

Als Ausgangsstoffe für das Herstellungsverfahren von Fettungsmitteln werden vorzugsweise höhere Fettsäuren oder Ester höherer Fettsäuren mit Kettenlängen im Bereich von $C_8$ bis $C_{24}$, vorzugsweise im Bereich von $C_{10}$ bis $C_{20}$, mit Jodzahlen von 10 bis 120 eingesetzt. Bevorzugt werden Gemische von Fettsäuren aus natürlich vorkommenden Fetten oder Ölen mit einem Anteil an einfach oder mehrfach ungesättigten Fettsäuren. Beispiele hierfür sind die aus Kokosöl, Sojaöl, Palmkernöl, Baumwollsaatöl, Rüböl, Leinöl, Rizinusöl, Sonnenblumenöl, Olivenöl, Klauenöl, Erdnussöl, Heringstran, Dorschtran, Haifischtran, Waltran, Talgfetten oder Schweineschmalz gewonnenen Fettsäuregemische. Auch die entsprechenden natürlich vorkommenden Fette oder Öle sowie natürlich vorkommende Wachsester, z.B. Spermöl, kommen als Ausgangsmaterial für das erfindungsgemässe Herstellungsverfahren von Fettungsmitteln in Betracht. Doch ist hier die Durchführung der Chlorierungs- bzw. Sulfochlorierungsreaktion häufig durch die hohe Viskosität der entsprechenden Produkte behindert.

Besonders bevorzugt als Ausgangsstoffe für das erfindungsgemässe Herstellungsverfahren von Fettungsmitteln sind synthetisch hergestellte Ester aus Gemischen gesättigter und ungesättigter Fettsäuren der Kettenlängen $C_8$ bis $C_{24}$, vorzugsweise $C_{10}$ bis $C_{20}$, mit Jodzahlen von 10–120, beispielsweise Decancarbonsäure, Palmitinsäure, Stearinsäure, Behensäure, Dodecencarbonsäure, Ölsäure, Linolsäure oder durch Paraffinoxidation hergestellte Carbonsäure, und einwertigen aliphatischen Alkoholen der Kettenlängen $C_1$ bis $C_4$, wie Methanol, Äthanol, Isopropanol, Butanol. Wegen ihrer leichten Zugänglichkeit werden die aus natürlichen tierischen oder pflanzlichen Fetten, Ölen oder Wachsen hergestellten Umesterungsprodukte mit den niederen einwertigen aliphatischen Alkoholen, insbesondere Methylalkohol, erhaltenen Fettsäureester bevorzugt. Schliesslich kommen auch Veresterungsprodukte der genannten Fettsäuren und mehrwertigen aliphatischen Alkoholen der Kettenlängen $C_2$ bis $C_6$, wie Äthylenglykol, 1,2-Propylenglykol, Glycerin, Pentaerythrit oder Sorbit, oder mit höheren Alkoholen der Kettenlängen $C_8$ bis $C_{24}$, wie Decylalkohol oder Oleylalkohol, in Betracht.

Die genannten Ausgangsstoffe werden in an sich bekannter Weise (a) zunächst bei niederen Temperaturen chloriert, wobei man die einzuleitende Chlormenge so bemisst, dass sie ausreicht, den olefinischen Anteil der Ausgangsstoffe durch additive Chlorierung abzusättigen. Man arbeitet bei Temperaturen bis höchstens 30 °C, vorzugsweise bei 20–25 °C, ohne Bestrahlung durch aktinisches Licht. In der Verfahrensstufe (b) wird sulfochloriert durch gleichzeitiges Einleiten von $Cl_2$ und $SO_2$ im Molverhältnis von 60:1 bis 1,4:1. Die Reaktionstemperatur beträgt 20–90 °C und wird gegebenenfalls durch Kühlen auf dem gewünschten Stand gehalten. Bevorzugt wird eine Reaktionstemperatur von 40–75 °C. Durch Bestrahlung mit UV-Licht (Quecksilberdampf-Lampe) wird der Reaktionsablauf gefördert. Die Reaktion ist nach etwa 2–10 Stunden beendet, nach welcher Zeit 5–30 Gewichtsprozent Chlor und 1–20 Gewichtsprozent $SO_2Cl$-Gruppen angelagert worden sind. Das Verhältnis von Chloratomen zu $SO_2Cl$-Gruppen liegt bei 0,7:1 bis 70:1. Vorzugsweise beträgt das Verhältnis 2:1 bis 20:1, insbesondere 3:1 bis 7:1. Die nachfolgende Verseifung wird mit wässriger, etwa 30%iger Natrium- oder Kaliumhydroxidlösung bei etwa 50 °C durchgeführt, die Neutralisation erfolgt entweder mit einem Überschuss der gleichen Alkalihydroxyde oder mit Ammoniaklösung oder mit einem aliphatischen oder cycloaliphatischen Amin oder einem Alkanolamin der Kettenlängen $C_2$ bis $C_6$, wie Triäthanolamin. Man erhält flüssige hochkonzentrierte wasseremulgierbare Produkte mit ausgezeichneter Oxidations-, Licht- und Säurestabilität, die sich für die Fettung aller hellen, pastellgefärbten und weissen Leder sowie zur Fettung auch wertvoller und empfindlicher Pelze hervorragend eignen.

Bei Verwendung dunkelfarbiger oder stärker ungesättigter Ausgangsstoffe kann sich eine Bleichung der Sulfonierungsprodukte empfehlen. Diese wird in üblicher Weise durch Zugabe geringer Mengen von etwa 0,5 bis 5%, vorzugsweise 1 bis 4% $H_2O_2$ zum sauren Sulfonierungsprodukt durchgeführt, wobei die Temperaturen zwischen 20 und 80, vorzugsweise zwischen 40 und 60 °C liegen. Durch diese Massnahme können auch dunkelfarbige Sulfonierungsprodukte sehr weitgehend aufgehellt werden.

Die Produkte werden in üblicher Weise in Form wässriger Emulsionen zum Lickern von Leder oder zur Pelzbehandlung angewendet. Die Produkte sind selbstemulgierend, so dass weitere Zusätze an Emulgatoren im allgemeinen nicht notwendig sind. Zur Erzielung spezieller Effekte können die Sulfonierungsprodukte jedoch mit den entsprechenden nicht sulfonierten Chlorierungsprodukten, oder anderen üblichen Lederbehandlungsmitteln kombiniert werden, wie z.B. nichtsulfonierte Öle oder Fette, wie Fischtran, Spermöl, Klauenöl und dergleichen oder synthetische Fettungsmittel, wie Chlorparaffine, Paraffinsulfonate, sulfierte native Fette oder Öle oder synthetische Fettsäureester, oder Mineralöle oder dergleichen, gegebenenfalls in Verbindung mit anionischen, nichtionogenen oder kationischen Emulgatoren, wie Ethylenoxidanlagerungsprodukte an höhere Fettalkohole, Alkylphenole oder Fettamine der Kettenlängen $C_{10}$ bis $C_{20}$. Eine Stabilisierung der Produkte kann durch Unschädlichmachen evtl. noch vorhandener oder neu gebildeter Chlorwasserstoffreste mittels Epoxidverbindungen in Mengen von 0,5 bis 5 Gewichtsprozent erreicht werden. Beispiele hierfür sind: Glyzid, Epichlorhydrin, Glyzidylether ein- oder mehrwertiger Alkohole, wie Glykol, Glycerin oder Sorbit, sowie epoxidierte Fettstoffe, z.B. epoxidiertes Sojaöl, Leinöl oder Ölsäurebutylester.

Die Produkte werden vom Leder gut aufgenommen und geben ausgezeichnete Fettungs- und Weichmachungseffekte, die eine bemerkenswerte Beständigkeit gegen Wasser und wässrige oder organische Reinigungsmittellösungen besitzen. Ihre Neigung zum Wandern bei thermischer Beanspruchung ist sehr gering, so dass sich z.B. Verklebungsvorgänge oder das Anvulkanisieren von Gummisohlen an Schuhoberteile ohne Schwierigkeiten durchführen lassen. Besonders hervorzuheben ist die gute Licht-, Oxidations- und Säurebeständigkeit der Produkte, die sie auch zur Fettung empfindlicher und heller Leder und Pelze geeignet machen. Die behandelten Leder oder Pelze zeichnen sich durch einen besonders angenehmen weichen und schmalzigen Griff sowie einen schönen Glanz des Pelzhaares aus.

Die in den nachfolgenden Beispielen beschriebenen Chlorierungs- und Sulfochlorierungsreaktionen wurden in der folgenden Apparatur durchgeführt:

Auf einem 2-1-Rundkolben mit Bodenablass steht ein Reaktorturm aus Glas, der mit Raschigringen gefüllt ist und der einen Doppelmantel für eine Heiz- bzw. Kühlflüssigkeit besitzt.

Das Einsatzmaterial wird durch den Bodenablass des Rundkolbens mittels einer Schlauchpumpe durch ein beheizbares Steigrohr in den Kopf des Reaktionsturmes gepumpt.

Die Gase (Chlor und Schwefeldioxid) werden am unteren Ende des Turmes über Nadelventile eingeleitet. Die Dosierung wird mittels Rotametern durchgeführt. Die Strömungsgeschwindigkeit der Gase liegt für Chlor zwischen 25 und 120 l/h und für $SO_2$ zwischen 4 und 50 l/h.

Das enstandene HCl-Gas wird mit dem Rest an nicht umgesetzten Ausgangsgasen am Kopf des Reaktionsrohres über ein Waschflaschensystem entnommen. Die gläserne Reaktionskolonne kann von aussen mit einer Hg-Dampflampe bestrahlt werden.

Beispiel 1

1000 g Talgfettsäuremethylester ($C_{16}$–$C_{18}$; J. Z. 53) wurden im Reaktor vorgelegt und während 1 Stunde bei 25 °C mit 50 ml Chlor begast.

Danach wurde auf 50 °C aufgeheizt und unter Bestrahlung mit einer Quecksilberdampf-Lampe 50 l/h Chlor und 12,5 l/h $SO_2$ eingeleitet. Während der Reaktion wurde der Kolbeninhalt mittels einer Schlauchpumpe umgepumpt. Die Reaktion wurde nach 3 Stunden beendet und das Reaktionsprodukt (1399 g) im Vakuum von den gelö-

sten Gasen (hauptsächlich HCl) befreit. Es enthielt 22.85 Gew.-% gebundenes Chlor und 8,90 Gew.-% gebundenes $SO_2Cl$. Die Jodzahl lag unter 1. Das in verdünnter NaOH aufgefangene Abgas bestand aus 193,1 g HCl, 8,54 g $SO_2$ und 0,6 g $Cl_2$.

Die Verseifung und Neutralisation wurde mit 300 g wässriger 30%iger NaOH bei Temperaturen von 40–50 °C durchgeführt. Dabei entstand eine stabile Emulsion, die ca .40% Chlor-Talgfettsäuremethylestersulfonat, 42% Chlor-Talgfettsäuremethylester und 18% anorganische Salze und Wasser enthielt.

Beispiel 2

In einer ähnlichen, aber grösseren Umlauf-Apparatur wie in Beispiel 1 wurden 17,25 kg Talgfettsäuremethylester vorgelegt, auf 25 °C erwärmt und binnen einer Stunde 1000 l Chlor eingeleitet, wobei die Temperatur auf 25 °C gehalten wurde.

Danach wurde das Reaktionsgemisch auf 50 °C erwärmt und 500 l/h Chlor und 150 l/h $SO_2$ unter Bestrahlung mit einer Quecksilberdampflampe 4 Stunden lang eingeleitet. Die Reaktion lief unter Entwicklung von HCl ab, das abgeführt und in verdünnter NaOH aufgefangen wurde. Nach Beendigung der Sulfochlorierung wurde das Produkt unter Vakuum entgast. Die Ausbeute betrug 23,7 kg Chlor-Talgfettsäuremethyleser-sulfonat mit 20,85 Gew.-% gebundenem Chlor und 10,50 Gew.-% gebundenem $SO_2Cl$, Jodzahl unter 1. Im Abgas waren enthalten: 3,0 kg HCl, 0,2 kg $SO_2$ und 0,0 kg Chlor.

1151 g des Reaktionsproduktes wurden mit 268 g einer 31%igen Natriumhydroxidlösung verseift und neutralisiert. Das homogenisierte Produkt enthielt ca. 50 Gew.-% Chlortalgfettsäuremethylester-sulfonat, 30 Gew.-% Chlortalgfettsäuremethylester und 20 Gew.-% anorganische Salze und Wasser. Das Produkt liess sich in Wasser zu einer stabilen Emulsion verdünnen.

Ein Vergleichsversuch mit gleichen Mengen Talgfettsäuremethylester und gleichen Mengen an Reaktionsgasen, bei dem jedoch das Gasgemisch 4 Stunden lang gleichzeitig und gleichmässig bei 50 °C eingeleitet worden war (750 l/h Chlor und 150 l/h $SO_2$) hatte folgendes Ergebnis: 22,9 kg Chlor-Talgfettsäuremethylester-sulfonat mit 20,85 Gew.-% gebundenem Chlor und 7,70 Gew.-% gebundenem $SO_2Cl$, Jodzahl unter 1. Im Abgas waren enthalten: 2,67 kg HCl, 0,67 kg $SO_2$ und 0,74 kg $Cl_2$.

Beispiel 3

In der gleichen Apparatur wie in Beispiel 2 verwendet, und unter den gleichen Bedingungen, wurden 17,25 kg Kokosfettsäuremethylester-Nachlauf ($C_{16}$–$C_{18}$, J. Z. 52) chloriert und sulfochloriert. Verbraucht wurden insgesamt 3000 l Chlor und 600 l $SO_2$. Es resultierten 23,5 kg Chlor-Kokosfettsäuremethylester-sulfonat mit 20,7 Gew.-% gebundenem Chlor und 8,70 Gew.-% gebundenem $SO_2Cl$, Jodzahl unter 1. Im Abgas waren enthalten: 2,6 kg HCl, 0,39 kg $SO_2$ und 0,41 kg $Cl_2$.

Beispiel 4

Ein mit synthetischen oder vegetabilen Gerbstoffen oder Harzgerbstoffen oder mit einer Kombination der genannten Gerbstoffe nachgegerbtes Schuhoberleder wurde im Fass bei 60 °C 45 Minuten bei 100–120% Flotte und 5 bis 6% des Sulfochlorierungsproduktes nach Beispiel 2 als fettende Substanz, auf Leder bezogen, gelickert. Die in üblicher Weise getrockneten und fertiggestellten Leder zeichneten sich durch weichen, geschmeidigen und vollen Griff, gute Narbenfestigkeit sowie ausgezeichnete Licht- und Oxidationsbeständigkeit aus.

Beispiel 5

Chromgegerbtes und gefärbtes Bekleidungsleder wurde bei 60 °C 45 Minuten mit 100–120% Flotte, 7–10% fettender Substanz, bestehend aus einem Gemisch von 80 Gew.-% des Sulfochlorierungsproduktes nach Beispiel 1 und 20 Gew.-% eines entsprechenden nicht sulfierten Chlor-Talgfettsäuremethylesters, im Fass gelickert. Die in üblicher Weise getrockneten und fertiggestellten Leder zeichneten sich durch weichen, geschmeidigen und vollen Griff sowie durch ausgezeichnete Licht- und Oxidationsstabilität aus.

Beispiel 6

Pastellgefärbte, chromgegerbte Handschuhleder aus Lammfellen wurden bei 60 °C 45 Minuten mit 100% Flotte und 6–8% fettender Substanz, bestehend aus einem Gemisch aus 75% des Sulfochlorierungsproduktes nach Beispiel 3, 21% eines nicht sulfierten Chlor-Kokosfettsäuremethylesters und 4% eines mit 4 Mol Ethylenoxid umgesetzten Talgamins im Fass gelickert, getrocknet und in üblicher Weise fertiggestellt. Die Leder zeichneten sich durch zügigen, geschmeidigen, weichen Griff und gute Lichtbeständigkeit aus.

Beispiel 7

In üblicher Weise gepickelte Rindsblössen wurden mit 8% eines handelsüblichen Chromgerbstoffes mit einem $CR_2O_3$-Gehalt von 25% mit 100% Flotte gegerbt und im gleichen Bad mit 2% fettender Substanz, bestehend aus einem Gemisch aus 95% des Sulfochlorierungsproduktes nach Beispiel 1 und 5% eines mit 20 Mol Ethylenoxid umgesetzten Talgalkohols vorgelickert. Die in üblicher Weise nachgegerbten, gefärbten und mit 8–10% des Sulfochlorierungsproduktes nachgefetteten und getrockneten Möbelleder zeichneten sich durch einen geschmeidigen, weichen Griff und gute Lichtbeständigkeit aus.

**Patentansprüche**

1. Verfahren zur Herstellung von Fettungsmitteln für Leder oder Pelze durch Umsetzung von Gemischen gesättigter und ungesättigter Fettsäuren mit Kettenlängen im Bereich von $C_8$ bis $C_{24}$ oder Estern solcher Fettsäuren mit Chlor und $SO_2$ durch

a) Sulfochlorierung solcher Fettsäuren bzw. deren Ester mittels Chlor und $SO_2$ bei 20 bis 90 °C, gegebenenfalls unter UV-Bestrahlung, und

b) anschliessender Verseifung der erhaltenen Produkte zu den entsprechenden Alkali-, Ammonium- oder Aminsalzen, dadurch gekennzeichnet, dass man

c) solche Fettsäuren bzw. deren Ester vor der Sulfochlorierung bis zur Absättigung der Doppelbindungen bei Temperaturen bis maximal 30 °C, gewünschtenfalls bei Temperaturen zwischen 20 und 25 °C, chloriert, wobei

d) die nach Chlorierung und Sulfochlorierung erhaltenen Produkte einen Gehalt an organisch gebundenem Chlor zwischen 5 und 30 Gew.-% und einen Gehalt an $SO_2Cl$-Gruppen zwischen 1 und 20 Gew.-% sowie ein Verhältnis von organisch gebundenen Chloratomen zu $SO_2Cl$-Gruppen im Bereich von 0,7:1 bis 70:1 aufweisen.

2. Verfahren nach Anspruch 1, dadurch gekennzeichnet, dass man für die Umsetzung Fettsäuren aus natürlich vorkommenden Fetten oder Ölen mit Kettenlängen im Bereich von $C_{10}$ bis $C_{20}$ mit Jodzahlen von 10 bis 120 einsetzt.

3. Verfahren nach Anspruch 1, dadurch gekennzeichnet, dass man für die Umsetzung Methylester von Fettsäuren mit Kettenlängen im Bereich von $C_{10}$ bis $C_{20}$ und Jodzahlen von 10 bis 120 einsetzt.

4. Verfahren nach den Ansprüchen 1 bis 3, dadurch gekennzeichnet, dass die nach Chlorierung und Sulfochlorierung erhaltenen Produkte ein Verhältnis von organisch gebundenen Chloratomen zu $SO_2Cl$-Gruppen im Bereich von 2:1 bis 20:1, vorzugsweise im Bereich von 3:1 bis 7:1, aufweisen.

## Revendications

1. Procédé pour la réalisation de produits pour nourrir des cuirs ou des peaux, par transformation de mélange d'acides gras saturés et non-saturés avec des longueurs de chaînes allant de $C_8$ à $C_{24}$ ou d'esters de ces acides gras avec du chlore et du $SO_2$ par:

a) sulfochlorage de ces acides ou de leur ester par du chlore et du $SO_2$ entre 20 et 90°C, éventuellement sous rayonnement ultra-violet, et

b) ensuite, saponification des produits obtenus pour donner des sels alcalins, les sels d'ammonium ou les sels aminés correspondants, procédé caractérisé en ce que:

c) on chlore ces acides gras ou leur ester avant le sulfochlorage jusqu'à saturation des liaisons doubles à des températures atteignant au maximum 30 °C et, dans le cas le plus souhaitable, à des températures comprises entre 20 et 25 °C,

d) les produits obtenus après chlorage et sulfochlorage présentant une teneur en chlore organiquement lié comprise entre 5 et 30% en poids et une teneur en groupes $SO_2Cl$ comprise entre 1 et 20% en poids, ainsi qu'un rapport des atomes de chlore organiquement liés aux groupes $SO_2Cl$ de l'ordre de 0,7:1 à 70:1.

2. Procédé selon la revendication 1, caractérisé en ce qu'on met en œuvre pour la transformation des acides gras issus de graisses ou d'huiles de provenance naturelle avec des longueurs de chaînes de l'ordre de $C_{10}$ à $C_{20}$ avec des indices d'iode de 10 à 120.

3. Procédé selon la revendication 1, caractérisé en ce qu'on met en œuvre pour la transformation des esters méthyliques d'acides gras avec des longueurs de chaînes de l'ordre de $C_{10}$ à $C_{20}$ et des indices d'iode de 10 à 120.

4. Procédé selon les revendications 1 à 3, caractérisé en ce que les produits obtenus après chlorage et sulfochlorage ont un rapport des atomes de chlore organiquement liés aux groupes $SO_2Cl$ de l'ordre de 2:1 à 20:1, de préférence de l'ordre de 3:1 à 7:1.

## Claims

1. A process for the production of fat-liquoring agents for leather or skins by reaction of mixtures of saturated and unsaturated fatty acids having chain lenghts of from $C_8$–$C_{24}$ or esters thereof with chlorine and $SO_2$ by

a) sulfochlorination of these fatty acids or esters thereof with chlorine and $SO_2$ at 20 to 90 °C, optionally under UV irradiation, and

b) subsequent saponification of the products obtained to the corresponding alkali, ammonium or amine salts, characterized in that

c) before sulfochlorination, the fatty acids or esters thereof are chlorinated at temperatures of up to at most 30 °C and, if desired, at temperatures of from 20 to 25 °C to saturation of the double bonds,

d) the products obtained after chlorination and sulfochlorination having an organically bound chlorine content of from 6 to 30% by weight and an $SO_2Cl$-group content of from 1 to 20% by weight for a ratio of organically bound chlorine atoms to $SO_2Cl$ groups of from 0.7:1 to 70:1.

2. A process as claimed in Claim 1, characterized in that fatty acids of naturally occurring fats or oils having chain lenghts of from $C_{10}$ to $C_{20}$ and iodine numbers of from 10 to 120 are used for the reaction.

3. A process as claimed in Claim 1, characterized in that methylesters of fatty acids having chain lenghts of from $C_{10}$ to $C_{20}$ and iodine numbers of from 10 to 120 are used for the reaction.

4. A process as claimed in Claims 1 to 3, characterized in that the products obtained after chlorination and sulfochlorination show a ratio of organically bound chlorine atoms to $SO_2Cl$ groups of from 2:1 to 20:1 and preferably of from 3:1 to 7:1.